# EUROPÄISCHE PATENTSCHRIFT

(11) **EP 3 494 109 B1**
(45) Veröffentlichungstag und Bekanntmachung des Hinweises auf die Patenterteilung: **23.10.2024**
(21) Anmeldenummer: 17749168.5
(22) Anmeldetag: 31.07.2017
(51) Int. Cl.: C07D 317/72

(54) **VERFAHREN ZUR HERSTELLUNG VON SPIROKETAL-SUBSTITUIERTEN CYCLISCHEN KETOENOLEN**
PROCESS FOR THE PREPARATION OF SPIROKETAL-SUBSTITUTED CYCLIC KETOENOLS
PROCÉDÉ POUR LA PRÉPARATION KETOENOLS CYCLIQUES SUBSTITUÉS PAR SPIROKETAL

(30) Priorität: 04.08.2016 EP 16182706
(43) Veröffentlichungstag der Anmeldung: 12.06.2019
(73) Patentinhaber: Bayer CropScience Aktiengesellschaft, 40789 Monheim am Rhein (DE)
(72) Erfinder: HIMMLER, Thomas, 51519 Odenthal (DE); HAHN, Julia, Johanna, 40589 Düsseldorf (DE); CREDE, Joachim, 42277 Wuppertal (DE)
(74) Vertreter: BIP Patents
(86) Internationale Anmeldenummer: PCT/EP2017/069287
(87) Internationale Veröffentlichungsnummer: WO 2018/024659

(56) Entgegenhaltungen:
- WO-A1-2007/096058
- WO-A2-2006/089633

## Beschreibung

Die Erfindung betrifft ein neues Verfahren zur Herstellung von spiroketal-substituierten cyclischen Ketoenolen, die als Insektizide, Akarizide oder Herbizide Verwendung finden können. Ebenfalls Gegenstand der vorliegenden Erfindung sind neue Zwischenprodukte zur Herstellung von spiroketal-substituierten cyclischen Ketoenolen.

Es ist bereits bekannt geworden, dass bestimmte spiroketal-substituierte cyclische Ketoenole insektizide, akarizide oder herbizide Wirksamkeit zeigen *(*WO 99/16748*;* WO 06/089633). Eine bekannt gewordene Synthese (A) solcher spiroketal-substituierter cyclischer Ketoenole geht von entsprechend spiroketal-substituierten Cyclohexanonen der allgemeinen Formel (I) aus, die in einer Bucherer-Bergs-Reaktion zu den spiroketal-substituierten Hydantoinen der allgemeinen Formel (II) umgesetzt werden. Alkalische Verseifung dieser Hydantoine ergibt die spiroketal-substituierten Aminosäuren der allgemeinen Formel (III). Diese Aminosäuren werden dann nach bekannten Methoden der organischen Chemie (beispielsweise durch Umsetzung mit einem Alkohol R⁷-OH und Thionylchlorid) zu den spiroketal-substituierten Aminosäureestern der allgemeinen Formel (IV; R⁷ gleich C₁-C₆-Alkyl) verestert. Diese Aminosäureester werden dann mit Phenylessigsäurechloriden der allgemeinen Formel (VII) am Stickstoff zu den Verbindungen der allgemeinen Formel (VIII) acyliert. Die Verbindungen der allgemeinen Formel (VIII) werden dann in einer Dieckmann-Reaktion durch Einwirkung einer starken Base wie Kalium-tertiärbutylat oder Natriummethanolat zu den spiroketal-substituierten cyclischen Ketoenolen der allgemeinen Formel (XI) cyclisiert. Dieses Verfahren (A) ist in Schema 1 abgebildet. Ein erheblicher Nachteil dieses Verfahrens (A) besteht darin, dass bei der Veresterung der Aminosäuren der allgemeinen Formel (III) unter sauren Bedingungen fast immer auch eine zumindest teilweise Umketalisierung des cyclischen Ketals zu einem acyclischen Ketal (geminale Bis(alkoxy)-Verbindung) der allgemeinen Formel (V) stattfindet. Außerdem zeigt sich in der Praxis, dass auch unter prinzipiell wasserfreien Bedingungen, wenn auch nur in geringeren Anteilen, zusätzlich Ketone der allgemeinen Formel (VI) gebildet werden können. Man erhält dann also den Ester als ein Gemisch aus zumindest zwei Produkten der allgemeinen Formeln (IV) und (V), das auch in den folgenden Stufen des Verfahrens (A) zu entsprechenden Produktgemischen führt. Zusätzlich führt die leichte Hydrolysierbarkeit der acyclischen Ketale dazu, dass auch N-acylierte Ketone der allgemeinen Formel (X) entstehen (identisch mit den Produkten der N-Acylierung der Ketoverbindungen der allgemeinen Formel (VI)). So erhält man also nach der N-Acylierung mit einem Phenylessigsäurechlorid der allgemeinen Formel (VII) die Amide der allgemeinen Formeln (VIII), (IX) und (X). Die Dieckmann-Cyclisierung führt dann zu einem Gemisch der cyclischen Ketoenole der allgemeinen Formeln (XI), (XII) und (XIII). Deshalb ist es für den Erhalt eines sauberen Produktes der allgemeinen Formel (XI) unter technischen Bedingungen (beispielsweise keine Reinigung der Zielverbindung durch Chromatographie möglich) unerlässlich, in einer zusätzlichen Stufe dieses Gemisch der Verbindungen der allgemeinen Formeln (XI), (XII) und (XIII) mit einem Diol der allgemeinen Formel (XIV) in Gegenwart eines sauren Katalysators zu der einheitlichen Verbindung der allgemeinen Formel (XI) umzusetzen. Dieser zusätzliche Schritt ist zeit- und kostenintensiv und unökonomisch.

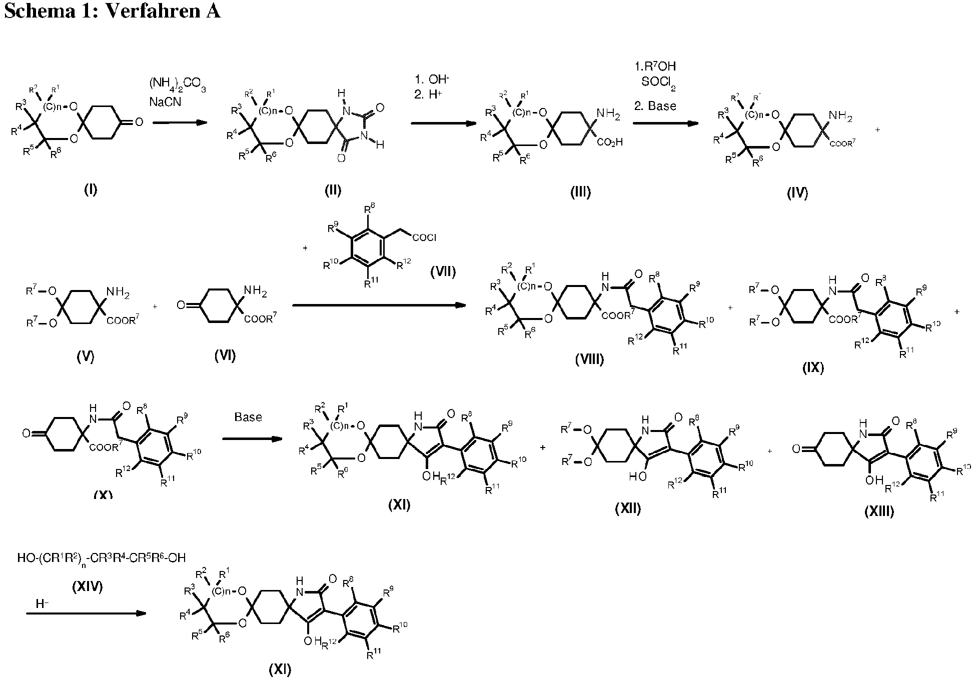

Es bestand daher Bedarf an einem einfacheren, kürzeren Verfahren zur Herstellung von spiroketal-substituierten cyclischen Ketoenolen der allgemeinen Formel (XI).

Es wurde nun gefunden, dass sich die Synthese von spiroketal-substituierten cyclischen Ketoenolen der allgemeinen Formel (XI) überraschend vereinfachen lässt, indem man die spiroketal-substituierten Aminosäuren der allgemeinen Formel (III) in einem ersten Schritt (1) unter Schotten-Baumann-Bedingungen mit einem Phenylessigsäurechlorid der allgemeinen Formel (VII) am Stickstoff acyliert und so die Amide der allgemeinen Formel (XV) erhält; im zweiten Schritt (2) des erfindungsgemäßen Verfahrens dann eine Veresterung unter sauren Bedingungen mit dem Diol der allgemeinen Formel (XIV) durchführt, wobei ein Gemisch der Ester der allgemeinen Formel (XVI) und Diester der allgemeinen Formel (XVII) erhalten werden kann; und im dritten Schritt (3) des erfindungsgemäßen Verfahrens dann die Dieckmann-Cyclisierung zu den spiroketal-substituierten cyclischen Ketoenolen der allgemeinen Formel (XI) durchführt. Das erfindungsgemäße Verfahren (B) ist in Schema 2 dargestellt.

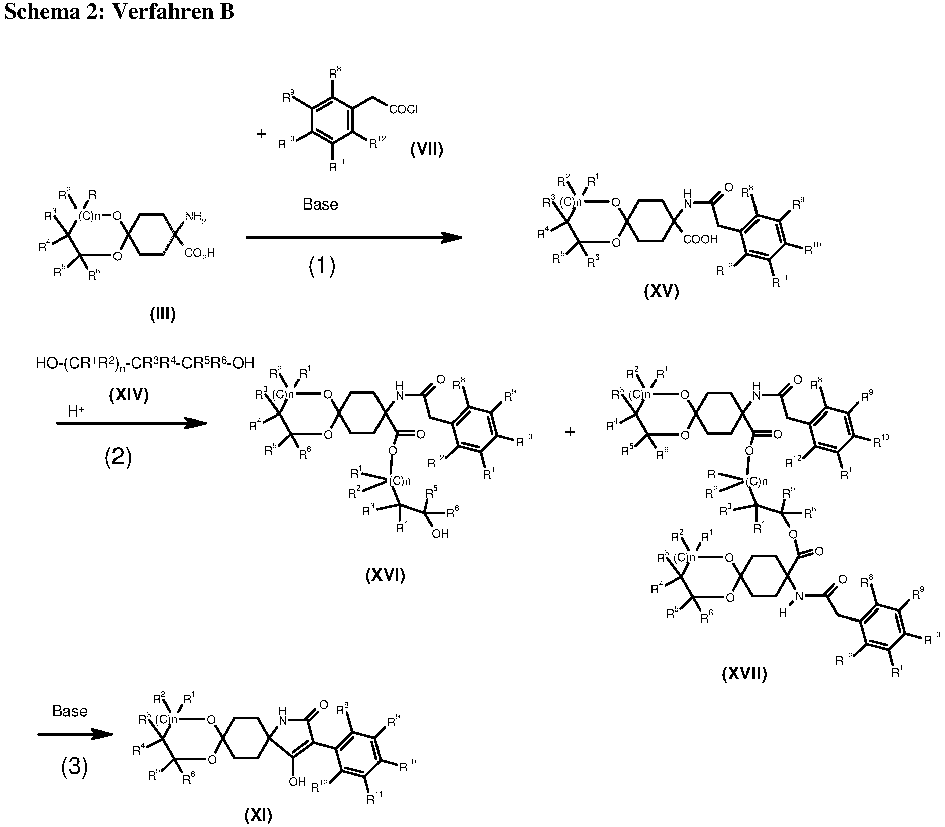

Die vorliegende Erfindung beinhaltet somit ein neues Verfahren (B) zur Herstellung von spiroketal-substituierten cyclischen Ketoenolen der allgemeinen Formel (XI), dadurch gekennzeichnet, dass in dem ersten Schritt (1) des Verfahrens spiroketal-substituierten Aminosäuren der allgemeinen Formel (III) 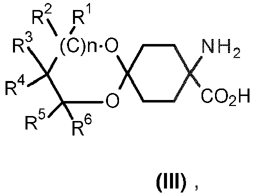 in der die Reste
- R¹ bis R⁶: unabhängig voneinander für Wasserstoff, Methyl, Ethyl oder Phenyl
stehen und
- n: für 0 oder 1
steht,
in Gegenwart einer Base mit einem Phenylessigsäurechlorid der allgemeinen Formel (VII) 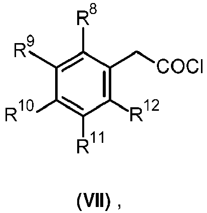
in der die Reste
R⁸ bis R¹² unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl
stehen,
zu Verbindungen der allgemeinen Formel (XV) 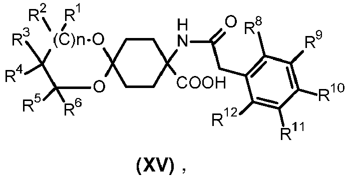
in der n und die Reste
R¹ bis R⁶ und R⁸ bis R¹² die oben angegebenen Bedeutungen haben,
umsetzt;
daraufhin in dem zweiten Schritt (2) des erfindungsgemäßen Verfahrens (B) eine Verbindung der allgemeinen Formel (XV) mit einem α, 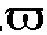 -Diol der allgemeinen Formel (XIV)

   HO-(CR¹R²)ₙ-CR³R⁴-CR⁵R⁶-OH **(XIV)**,
in der n und die Reste R¹ bis R⁶ die oben angegebenen Bedeutungen haben und im konkreten Einzelfall mit denjenigen in der Verbindung der allgemeinen Formel (XV) identisch sind,
in Gegenwart einer Säure als Katalysator zu den Mono- und Diestern der allgemeinen Formeln (XVI) und (XVII) 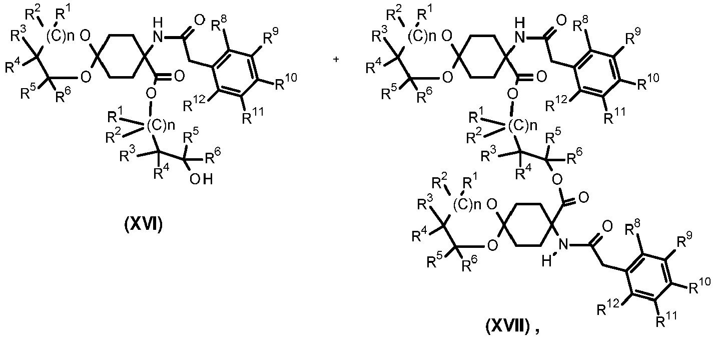
in denen n und die Reste R¹ bis R⁶ und R⁸ bis R¹² die oben angegebenen Bedeutungen haben und sowohl n als auch die sich entsprechenden Reste R¹ bis R⁶ und R⁸ bis R¹² an beiden Positionen der Verbindungen der allgemeinen Formeln (XVI) oder (XVII) identisch sind,
verestert;
daraufhin in dem dritten Schritt (3) des erfindungsgemäßen Verfahrens (B) die Verbindungen der allgemeinen Formeln (XVI) und (XVII), in denen n und die Reste R¹ bis R⁶ und R⁸ bis R¹² die oben angegebenen Bedeutungen haben,
durch Reaktion mit einer starken Base zu den spiroketal-substituierten cyclischen Ketoenolen der allgemeinen Formel (XI) 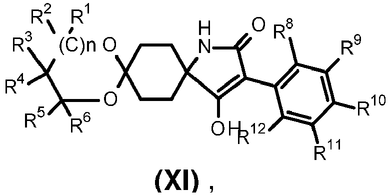
in der n und die Reste R¹ bis R⁶ und R⁸ bis R¹² die oben angegebenen Bedeutungen haben,
umsetzt.
R¹ bis R⁶ bedeutet R¹, R², R³, R⁴, R⁵, R⁶.
R⁸ bis R¹² bedeutet R⁸, R⁹, R¹⁰, R¹¹, R¹².
In den Formeln (III), (VII), (XV), (XIV), (XVI), (XVII), (XI) steht
R¹ bis R⁶ unabhängig voneinander bevorzugt für Wasserstoff, Methyl oder Ethyl,
R⁸ bis R¹² unabhängig voneinander bevorzugt für Wasserstoff, Methyl, Ethyl, Fluor, Chlor, Methoxy, Ethoxy, Trifluormethoxy oder gegebenenfalls durch Methyl, Ethyl, Methoxy, Ethoxy, Fluor, Chlor oder Brom substituiertes Phenyl,
n bevorzugt für 0 oder 1;
R¹ bis R⁶ unabhängig voneinander besonders bevorzugt für Wasserstoff oder Methyl,
R⁸ bis R¹² unabhängig voneinander besonders bevorzugt für Wasserstoff, Methyl, Ethyl, Fluor, Chlor, Methoxy, Ethoxy oder gegebenenfalls durch Methyl, Ethyl, Methoxy, Ethoxy, Fluor oder Chlor substituiertes Phenyl,
n besonders bevorzugt für 0 oder 1;
R³ bis R⁶ unabhängig voneinander ebenso besonders bevorzugt für Wasserstoff oder Methyl,
R⁸ bis R¹² unabhängig voneinander ebenso besonders bevorzugt für Wasserstoff, Methyl, Ethyl, Fluor, Chlor, Methoxy, Ethoxy oder gegebenenfalls durch Methyl, Methoxy, Fluor oder Chlor substituiertes Phenyl,
n ebenso besonders bevorzugt für 0;
R³ bis R⁶ unabhängig voneinander ganz besonders bevorzugt für Wasserstoff oder Methyl,
R⁸ bis R¹² unabhängig voneinander ganz besonders bevorzugt für Wasserstoff, Methyl oder Chlor,
n ganz besonders bevorzugt für 0.

Hervorgehoben steht n für 0, R³ bis R⁶ für Wasserstoff, R⁸ für Methyl, R⁹ für Wasserstoff, R¹⁰ für Chlor, R¹¹ für Wasserstoff, R¹² für Methyl.

Hervorgehoben steht n für 0, R³ bis R⁶ für Wasserstoff, R⁸ für Methyl, R⁹ für Wasserstoff, R¹⁰ für Methyl, R¹¹ für Wasserstoff, R¹² für Methyl.

Hervorgehoben steht n für 0, R³ für Wasserstoff, R⁴ für Methyl, R⁵ für Wasserstoff, R⁶ für Methyl, R⁸ für Methyl, R⁹ für Wasserstoff, R¹⁰ für Wasserstoff, R¹¹ für Mehyl, R¹² für Wasserstoff.

Hervorgehoben steht n für 1, R¹ für Wasserstoff, R² für Wasserstoff, R³ für Methyl, R⁴ für Methyl, R⁵ für Wasserstoff, R⁶ für Wasserstoff, R⁸ für Methyl, R⁹ für Wasserstoff, R¹⁰ für Chlor, R¹¹ für Wasserstoff, R¹² für Methyl.

Hervorgehoben steht n für 1, R¹ bis R⁶ für Wasserstoff, R⁸ für Methyl, R⁹ für Wasserstoff, R¹⁰ für Chlor, R¹¹ für Wasserstoff, R¹² für Methyl.

In den Formeln (I), (II) und (XIII), die in Verfahren A beschrieben sind, haben R¹, R², R³, R⁴, R⁵, R⁶, R⁸, R⁹, R¹⁰, R¹¹, R¹² und n die oben genannten Bedeutungen.

In den Formeln (IV), (VIII), (IX), (X) und (XII), die in Verfahren A beschrieben sind, haben R¹, R², R³, R⁴, R⁵, R⁶, R⁸, R⁹, R¹⁰, R¹¹, R¹² und n die oben genannten Bedeutungen und R⁷ steht für C₁-C₆-Alkyl. Besonders hervorgehoben steht n für 0; R³ bis R⁶ stehen für Wasserstoff; R⁸ und R¹² stehen für Methyl; R⁹ und R¹¹ stehen für Wasserstoff; R¹⁰ steht für Chlor und R⁷ steht für Methyl:
Verbindung der Formel (IV-1): 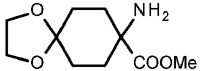
Verbindung der Formel (VIII-1): 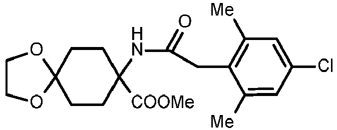
Verbindung der Formel (IX-1): 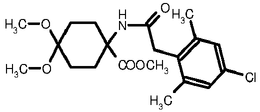
Verbindung der Formel (X-1): 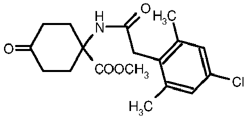
Verbindung der Formel (XII-1): 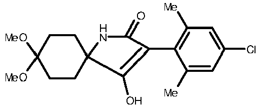 In den Formeln (V) und (VI), die in Verfahren A beschrieben sind, steht R⁷ für C₁-C₆-Alkyl. Besonders hervorgehoben steht R⁷ steht für Methyl:
Verbindung der Formel (V-1): 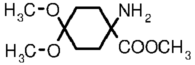
Verbindung der Formel (VI-1): 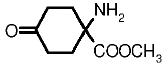 (Me = CH₃ = Methyl)

Die erste Stufe (1) des erfindungsgemäßen Verfahrens (B) wird in einem unter den Reaktionsbedingungen inerten Lösungsmittel durchgeführt. Als Lösungsmittel kommen beispielsweise in Frage: Dichlormethan, Toluol, ortho-, meta oder para-Xylol, Mesitylen, Chlorbenzol, ortho-Dichlorbenzol, Acetonitril, Butyronitril, N,N-Dimethylformamid, N,N-Dimethylacetamid, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, Essigsäureethylester, Essigsäurebutylester, Wasser oder Gemische dieser Lösungsmittel. Bevorzugt sind Toluol, ortho-, meta oder para-Xylol, Chlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, Essigsäureethylester, Essigsäurebutylester, Wasser oder Gemische dieser Lösungsmittel. Besonders bevorzugt sind Gemische von Toluol, ortho-, meta oder para-Xylol, Chlorbenzol, 2-Methyl-tetrahydrofuran, Cyclopentyl-methyl-ether, Methyl-tertiärbutyl-ether, Tertiäramyl-methyl-ether mit Wasser (sogenannte Schotten-Baumann-Bedingungen).

Als Base können in der ersten Stufe (1) des erfindungsgemäßen Verfahrens (B) organische Basen wie beispielsweise Trimethylamin, Triethylamin, Piperidin, Morpholin, Pyridin; oder anorganische Basen wie Ammoniak, Lithiumhydroxid, Natriumhydroxid, Kaliumhydroxid, Natriumcarbonat, Kaliumcarbonat, Natriumhydrogencarbonat, Kaliumhydrogencarbonat, Calciumcarbonat verwendet werden. Bevorzugt sind Natriumhydroxid und Kaliumhydroxid.

Feste anorganische Basen können als Feststoffe oder in Form ihrer wässrigen Lösungen eingesetzt werden. Bevorzugt verwendet man wässrige Lösungen.

Die Base wird üblicherweise in einer solchen Menge eingesetzt, dass sich ein pH-Wert zwischen 10 und 14 ergibt. Bevorzugt arbeitet man bei einem pH-Wert zwischen 11 und13.

Die erste Stufe (1) des erfindungsgemäßen Verfahrens (B) wird bei Temperaturen zwischen -5 und 50°C durchgeführt; bevorzugt zwischen 0 und 30°C.

Die Isolierung der Verbindungen der allgemeine Formel (XV) erfolgt nach bekannten üblichen Methoden der organischen Chemie wie Filtration, Phasentrennung oder Extraktion.

In der zweiten Stufe (2) des erfindungsgemäßen Verfahrens (B) können unter den Reaktionsbedingungen inerte Lösungsmittel verwendet werden. Als Lösungsmittel kommen beispielsweise in Frage: Dichlormethan, Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan oder Gemische dieser Lösungsmittel. Bevorzugt sind Toluol, ortho-, meta oder para-Xylol, Chlorbenzol, Acetonitril, Butyronitril, 2-Methyl-tetrahydrofuran, Cyclopentyl-methyl-ether, Methyl-tertiärbutyl-ether, Tertiäramyl-methyl-ether oder Gemische dieser Lösungsmittel.

Das α, 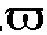 -Diol der allgemeinen Formel (XIV) wird in einer Menge von mindestens 0,5 Mol, bezogen auf 1 Mol der Verbindung der allgemeinen Formel (XV), eingesetzt. Es ist auch möglich, in einem beliebigen Überschuß an α, 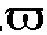 -Diol der allgemeinen Formel (XIV) zu arbeiten und dieses somit gleichzeitig als Lösungsmittel zu verwenden.

Die zweiten Stufe (2) des erfindungsgemäßen Verfahrens (B) wird in Gegenwart einer katalytischen Menge einer Säure durchgeführt. Als Säure kommen beispielsweise in Frage: Chlorwasserstoff, Schwefelsäure, Methansulfonsäure, Trifluormethansulfonsäure, para-Toluolsulfonsäure oder saure Ionenaustauscherharze wie beispielsweise Amberlite. Bevorzugt verwendet man Schwefelsäure oder para-Toluolsulfonsäure. Besonders bevorzugt verwendet man Schwefelsäure.

Die Säure wird in Mengen von 0,01 bis 20 Gewichtsprozent, bezogen auf die Verbindung der allgemeinen Formel (XV), eingesetzt. Bevorzugt sind 0,05 bis 10 Gewichtsprozent.

Die zweite Stufe (2) des erfindungsgemäßen Verfahrens (B) wird bei Temperaturen zwischen 20 und 150°C durchgeführt; bevorzugt zwischen 50 und 120°C.

Die Isolierung der Verbindungen der allgemeine Formeln (XVI) und (XVII) erfolgt nach bekannten üblichen Methoden der organischen Chemie wie Filtration, Phasentrennung oder Extraktion.

In der dritten Stufe (3) des erfindungsgemäßen Verfahrens (B) können unter den Reaktionsbedingungen inerte Lösungsmittel verwendet werden. Als Lösungsmittel kommen beispielsweise in Frage: Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, N,N-Dimethylformamid, N,N-Dimethylacetamid, N-Methyl-pyrrolidon, Methanol, Ethanol, 1-Butanol, Tertiärbutanol oder Gemische dieser Lösungsmittel. Bevorzugt sind N,N-Dimethylformamid, N,N-Dimethylacetamid, N-Methyl-pyrrolidon, Methanol, Tertiärbutanol oder Gemische dieser Lösungsmittel.

Als Basen können in der dritten Stufe (3) des erfindungsgemäßen Verfahrens (B) beispielsweise Natriumhydroxid, Kaliumhydroxid, Natriummethylat, Kaliummethylat, Natriumethylat, Kaliumethylat, Natrium-tertiärbutylat oder Kalium-tertiärbutylat verwendet werden. Bevorzugt sind Natriumhydroxid, Natriummethylat und Kalium-tertiärbutylat. Besonders bevorzugt verwendet wird Natriummethylat.

Die Basen werden in einer Menge von 0,9 bis 4 Moläquivalenten, bezogen auf die Verbindungen der allgemeinen Formeln (XVI) und (XVII) eingesetzt. Bevorzugt verwendet man 1 bis 3,5 Moläquivalente.

Die Temperatur im dritten Schritt (3) des erfindungsgemäßen Verfahrens (B) beträgt zwischen 20 und 150°C. Bevorzugt arbeitet man zwischen 40 und 100°C.

Die Isolierung der Verbindugen der allgemeinen Formel (XI) nach Einstellen des pH-Wertes des Reaktionsgemisches auf einen Wert zwischen 0 und 8 erfolgt nach bekannten üblichen Methoden der organischen Chemie wie Filtration, Phasentrennung oder Extraktion.

Die Verbindungen der Formel (III) sind teilweise bekannt (*WO 06*/*089633*), teilweise neu oder lassen sich nach den dort beschriebenen Verfahren herstellen.

Die Verbindungen der Formel (VII) sind teilweise bekannt (*WO 97*/*02243*), teilweise neu oder lassen sich nach den dort beschriebenen Verfahren herstellen.

Die Verbindungen der Formel (XIV) sind kommerziell erhältlich.

Ebenfalls Gegenstand der vorliegenden Erfindung ist die neue Verbindung 1-{[(4-Chlor-2,6-dimethylphenyl)acetyl] amino} -4-oxocyclohexancarbonsäure (XV-1)

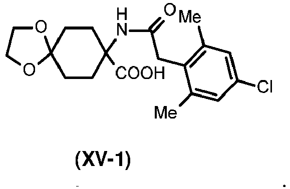

Die zur Herstellung der Verbindungen der allgemeinen Formel (XV) benötigten Aminosäuren der allgemeinen Formel (III) sind teilweise neu. Die bei ihrer Herstellung durch alkalische Hydrolyse der entsprechenden Hydantoine der allgemeinen Formel (II), gefolgt von Einstellen eines pH-Wertes von kleiner 7 und Abtrennen der anorganischen Salze, erzielten Ausbeuten sind nicht immer befriedigend. So beschreibt beispielsweise die *WO 06*/*089633* die Herstellung der Verbindung 8-Amino-1,4-dioxaspiro[4.5]decan-8-carbonsäure (III-1) durch Kochen der Verbindung 9,12-Dioxa-1,3-diazadispiro[4.2.4.2]tetradecan-2,4-dion (II-1) mit 9,8 Moläquivalenten Kaliumhydroxid in Form 30%iger Kalilauge. Anschließend wird mit konz. Salzsäure ein pH-Wert von 5,2 bis 5,3 eingestellt. Nach Filtration wird das Filtrat durch azeotrope Destillation mit Methanol auf etwa das halbe Volumen eingeengt. Kaliumchlorid wird abgesaugt und das Filtrat mit Methanol weiter azeotrop entwässert. Die isolierte Ausbeute an Zielverbindung beträgt nur 46% der Theorie. In einem weiteren bekannt gewordenen Verfahren *(*Journal ofAgricultural and Food Chemistry 2012 (60) 4779-4787*)* wird das Hydantoin (II-1) mit 7 Moläquivalenten Natriumhydroxid in Form von 3 N Natronlauge für 4 Tage unter Rückfluß gekocht. Anschließend wird auf 0°C gekühlt und der pH-Wert mit konz. Salzsäure auf 6 gestellt. Nach Filtration wird das Filtrat im Vakuum auf etwa ein Drittel des ursprünglichen Volumens eingeengt. Der ausgefallene Feststoff wurde abgesaugt und getrocknet. Die Ausbeute an Zielverbindung betrug nur 48% der Theorie.

Es bestand also Bedarf an einem verbesserten Verfahren zur Herstellung einer zur Herstellung der Verbindungen der allgemeinen Formel (XV^{#}) 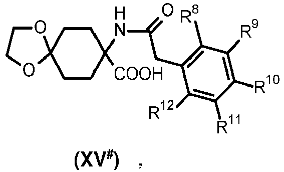
in der die Reste R⁸ bis R¹² die oben angegebenen Bedeutungen haben,
geeigneten Vorstufe.

Es wurde nun gefunden, dass sich anstelle der Aminosäure (III-1) ihre entsprechenden Natrium- oder Kaliumsalze der allgemeinen Formel (XIX) 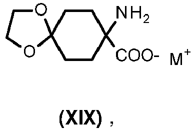 in der
- M: für Natrium oder Kalium
steht,
in einfacher Weise und hoher Ausbeute erhalten lassen.

Beschrieben wird ein neues Verfahren (C) zur Herstellung von Verbindungen der allgemeinen Formel (XIX), dadurch gekennzeichnet, dass man das Hydantoin der Formel (II-1, 9,12-Dioxa-1,3-diazadispiro[4.2.4.2]tetradecan-2,4-dion) mit Natronlauge oder Kalilauge unter Rückfluß erhitzt und anschließend die erhaltene Verbindung der allgemeinen Formel (XIX) durch Filtration isoliert.

Das Natrium- oder Kaliumhydroxid wird in Mengen von 1 bis 10 Moläquivalenten eingesetzt. Bevorzugt verwendet man zwischen 2 und 7 Äquivalente. Wird das Natrium- oder Kaliumhydroxid in diesen Mengen eingesetzt, fällt die Verbindung der Formel (XIX) als Feststoff aus.

Besonders bevorzugt verwendet man Natriumhydroxid.

Die Wassermenge beträgt zwischen 250 und 1500 mL pro Mol Hydantoin. Bevorzugt verwendet man zwischen 300 und 1000 mL pro Mol Hydantoin.

Die Reaktionstemperatur liegt zwischen 50 und 200°C. Bevorzugt arbeitet man zwischen 80 und 150°C.

Die Reaktion kann auch unter vermindertem oder erhöhtem Druck durchgeführt werden.

Die Verbindungen der allgemeinen Formel (XIX) werden durch einfache Filtration isoliert. Sie lassen sich nach analytischer Gehaltsbestimmung ohne weitere Aufreinigung anstelle der freien Aminosäuren in die Schotten-Baumann-Reaktion zur Herstellung der Verbindung (XV^{#}) einsetzen, wobei gleichzeitig auch ein Äquivalent Base eingespart wird, was einen weiteren Vorteil dieses Verfahrens darstellt.

Folgende Verbindungen der allgemeinen Formel (XIX) sind genannt 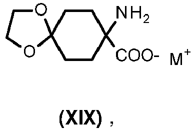 in der
- M: für Natrium oder Kalium
steht.

Bevorzugt ist die Verbindung der allgemeinen Formel (XIX), in der
- M: für Natrium
steht.

Ebenfalls Gegenstand der vorliegenden Erfindung ist ein neues Verfahren (D) zur Herstellung von Verbindungen der allgemeinen Formeln (XVI) und (XVII) 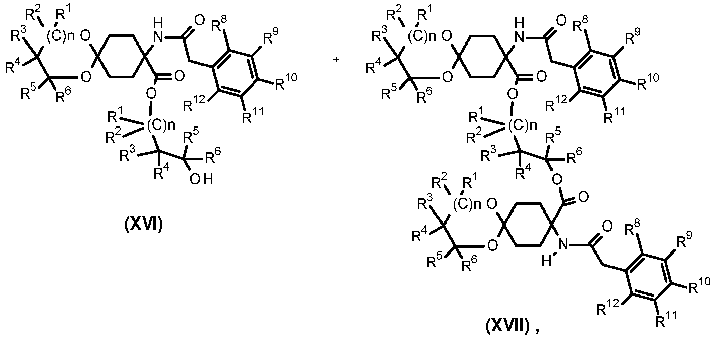 in denen die Reste
R¹ bis R⁶ und R⁸ bis R¹² die oben angegebenen Bedeutungen haben und die sich entsprechenden Reste R¹ bis R⁶ und R⁸ bis R¹² an beiden Positionen der Verbindungen der allgemeinen Formeln (XVI) oder (XVII) identisch sind, und
   - n: für 0 oder 1
steht und an beiden Positionen der Verbindungen der allgemeinen Formeln (XVI) oder (XVII) identisch ist,
dadurch gekennzeichnet, dass eine Ketoverbindung der allgemeinen Formel (XVIII) 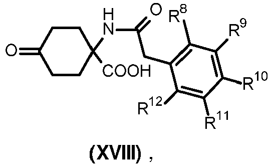
in der die Reste R⁸ bis R¹² die oben angegebenen Bedeutungen haben,
mit einem α, 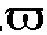 -Diol der allgemeinen Formel (XIV)

   HO-(CR¹R²)ₙ-CR³R⁴-CR⁵R⁶-OH (XIV),
in der n und die Reste R¹ bis R⁶ die oben angegebenen Bedeutungen haben und im konkreten Einzelfall mit denjenigen in den Verbindung der allgemeinen Formel (XVI) oder (XVII) identisch sind,
in Gegenwart einer Säure als Katalysator umsetzt.

Die im erfindungsgemäßen Verfahren (D) als Ausgangsstoffe verwendeten Verbindungen der allgemeinen Formel (XVIII) sind prinzipiell bekannt (*WO 06*/*089633*).

Im erfindungsgemäßen Verfahrens (D) können unter den Reaktionsbedingungen inerten Lösungsmittel verwendet werden. Als Lösungsmittel kommen beispielsweise in Frage: Dichlormethan, Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan oder Gemische dieser Lösungsmittel. Bevorzugt sind Toluol, ortho-, meta oder para-Xylol, Chlorbenzol, Acetonitril, Butyronitril, 2-Methyl-tetrahydrofuran, Cyclopentyl-methyl-ether, Methyl-tertiärbutyl-ether, Tertiäramyl-methyl-ether oder Gemische dieser Lösungsmittel.

Das α, 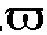 -Diol der allgemeinen Formel (XIV) wird in einer Menge von mindestens 2 Mol, bezogen auf 1 Mol der Verbindung der allgemeinen Formel (XVIII), eingesetzt. Es ist auch möglich, in einem beliebigen Überschuß an α, 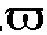 -Diol der allgemeinen Formel (XIV) zu arbeiten und dieses gleichzeitig als Lösungsmittel zu verwenden.

Das erfindungsgemäße Verfahren (D) wird in Gegenwart einer katalytischen Menge einer Säure durchgeführt. Als Säure kommen beispielsweise in Frage: Chlorwasserstoff, Schwefelsäure, Methansulfonsäure, Trifluormethansulfonsäure, para-Toluolsulfonsäure oder saure Ionenaustauscherharze wie beispielsweise Amberlite. Bevorzugt verwendet man Schwefelsäure oder para-Toluolsulfonsäure.

Die Säure wird in Mengen von 0,01 bis 20 Gewichtsprozent, bezogen auf die Verbindung der allgemeinen Formel (XVIII), eingesetzt. Bevorzugt sind 0,05 bis 10 Gewichtsprozent.

Das erfindungsgemäße Verfahren (D) wird bei Temperaturen zwischen 20 und 150°C durchgeführt; bevorzugt zwischen 50 und 120°C.

Die Isolierung der Verbindungen der allgemeine Formeln (XVI) und (XVII) erfolgt nach bekannten üblichen Methoden der organischen Chemie wie Filtration, Phasentrennung oder Extraktion.

Ebenfalls Gegenstand der vorliegenden Erfindung sind neue Verbindungen der allgemeinen Formeln (XVI) und (XVII) 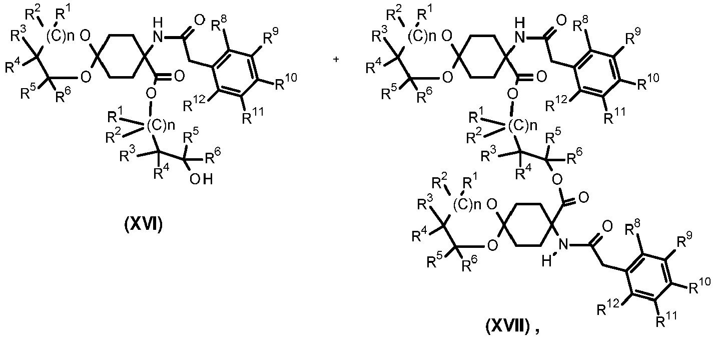 in denen die Reste
R¹ bis R⁶ unabhängig voneinander für Wasserstoff, Methyl, Ethyl oder Phenyl stehen und an beiden Positionen der Verbindungen der allgemeinen Formeln (XVI) oder (XVII) identisch sind,
   - n: für 0 oder 1
steht und an beiden Positionen der Verbindungen der allgemeinen Formeln (XVI) oder (XVII) identisch ist,
und die Reste
R⁸ bis R¹² unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl stehen und an beiden Positionen der Verbindungen der allgemeinen Formeln (XVI) oder (XVII) identisch sind.

Bevorzugt sind neue Verbindungen der allgemeinen Formeln (XVI) und (XVII), in denen die Reste
- R¹ bis R⁶: unabhängig voneinander für Wasserstoff, Methyl oder Ethyl
stehen und an beiden Positionen der Verbindungen der allgemeinen Formeln (XVI) oder (XVII) identisch sind,
- n: für 0 oder 1
steht und an beiden Positionen der Verbindungen der allgemeinen Formeln (XVI) oder (XVII) identisch ist
und die Reste
- R⁸ bis R¹²: unabhängig voneinander für Wasserstoff, Methyl, Ethyl, Fluor, Chlor, Methoxy, Ethoxy, Trifluormethoxy oder gegebenenfalls durch Methyl, Ethyl, Methoxy, Ethoxy, Fluor, Chlor oder Brom substituiertes Phenyl
stehen und an beiden Positionen der Verbindungen der allgemeinen Formeln (XVI) oder (XVII) identisch sind.

Besonders bevorzugt sind neue Verbindungen der allgemeinen Formeln (XVI) und (XVII), in denen die Reste
- R¹ bis R⁶: unabhängig voneinander für Wasserstoff oder Methyl
stehen und an beiden Positionen der Verbindungen der allgemeinen Formeln (XVI) oder (XVII) identisch sind,
- n: für 0
steht und die Reste
R⁸ bis R¹² unabhängig voneinander für Wasserstoff, Methyl, Ethyl, Fluor, Chlor, Methoxy, Ethoxy oder gegebenenfalls durch Methyl, Ethyl, Methoxy, Ethoxy, Fluor oder Chlor substituiertes Phenyl
stehen und an beiden Positionen der Verbindungen der allgemeinen Formeln (XVI) oder (XVII) identisch sind.

Ganz besonders bevorzugt sind
2-Hydroxyethyl-8-{[(4-chlor-2,6-dimethylphenyl)acetyl]amino}-1,4-dioxaspiro[4.5]decan-8-carboxylat ((XVI) mit n=0, R³ bis R⁶ gleich Wasserstoff, R⁸ und R¹² gleich Methyl, R⁹ und R¹¹ gleich Wasserstoff, R¹⁰ gleich Chlor).
Ethan-1,2-diyl-bis(8- { [(2,5-dimethylphenyl)acetyl] amino } -1,4-dioxaspiro [4.5] decan-8-carboxylat) ((XVII) mit n=0, R³ bis R⁶ gleich Wasserstoff, R⁸ und R¹² gleich Methyl, R⁹ und R¹¹ gleich Wasserstoff, R¹⁰ gleich Chlor).
3-Hydroxybutan-2-yl-8-{[(2,5-dimethylphenyl)acetyl]amino}-2,3-dimethyl-1,4-dioxaspiro[4.5]decan-8-carboxylat ((XVI) mit n=0, R³ und R⁵ gleich Wasserstoff, R⁴ und R⁶ gleich Methyl, R⁸ und R¹¹ gleich Methyl, R⁹, R¹⁰ und R¹² gleich Wasserstoff).
3-Hydroxy-2,2-dimethylpropyl-9- {[(4-chlor-2,6-dimethylphenyl)acetyl] amino} -3,3-dimethyl-1,5-dioxaspiro[5.5]undecan-9-carboxylat (((XVI) mit n=1, R¹, R², R⁵ und R⁶ gleich Wasserstoff, R³ und R⁴ gleich Methyl, R⁹ und R¹¹ gleich Wasserstoff, R⁸ und R¹² gleich Methyl, R¹⁰ gleich Chlor).

Die vorliegende Erfindung soll durch folgende Beispiele näher beschrieben werden, ohne durch diese eingeschränkt zu sein.

### Beispiel 1

### 2-Hydroxyethyl-8-{[(4-chlor-2,6-dimethylphenyl)acetyl]amino}-1,4-dioxaspiro[4.5]decan-8-carboxylat (Bsp. XVI-1) und Ethan-1,2-diyl-bis(8-{[(2,5-dimethylphenyl)acetyl]amino}-1,4-dioxaspiro[4.5]decan-8-carboxylat) (Bsp. (XVII-1)

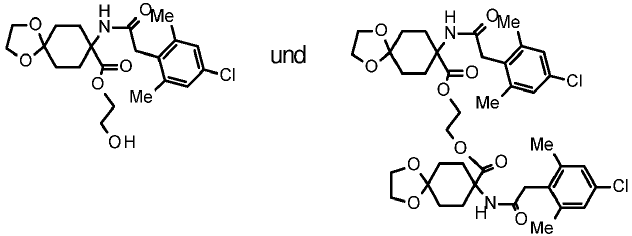

Zu einer Lösung von 3,82 g [10 mmol] 8-{[(4-Chlor-2,6-dimethylphenyl)acetyl]amino}-1,4-dioxaspiro[4.5]decan-8-carbonsäure in 15 ml Chlorbenzol gibt man 1,862 g [30 mmol] Ethylenglykol und 10 Tropfen konz. Schwefelsäure. Das Reaktionsgemisch wird 9 Stunden auf 88 bis 103°C erhitzt. Nach 5 Stunden werden weitere 8 Tropfen konz. Schwefelsäure zugesetzt. Das Reaktionsgemisch wird auf Raumtemperatur abgekühlt, mit 30 ml Chlorbenzol verdünnt und filtriert. Der Filterrückstand wird mit Petrolether gewaschen, in Methylenchlorid gelöst und mit gesättigter wässriger Natriumbicarbonatlösung und Wasser gewaschen. Nach Trocknen der organischen Phase über Natriumsulfat und Einengen im Vakuum erhält man 3,00 g farblosen Feststoff, der nach HPLC 68,8% 2-Hydroxyethyl-8-{[(4-chlor-2,6-dimethylphenyl)acetyl] amino }-1,4-dioxaspiro[4.5]decan-8-carboxylat (48,5% der Theorie) und 14,1% Ethan-1,2-diyl-bis(8- { [(2,5-dimethylphenyl)acetyl] amino } -1,4-dioxaspiro [4.5] decan-8-carboxylat) (10,7% d. Th.) enthält.

LC/MS (ESI positiv): m/e = 426 (MH⁺, 1 ³⁵Cl).

LC/MS (ESI positiv): m/e = 788 (MH⁺, 2 ³⁵Cl).

¹H-NMR (600 MHz, CDCl₃): δ = 1,25-1,35 (m; 2H), 1,6-1,7 (m; 2H), 1,8-1,9 (m; 2H), 2,05-2,15 (m; 2H), 2,24 (s; 6H), 2,9 (s,br; 1H), 3,51 (s; 2H), 3,7 (m, br; 2H), 3,85 (s; 4H), 4,24 (m; 2H), 5,48 (s; 1H), 7,04 (s; 2H) ppm.

¹H-NMR (600 MHz, CDCl₃): δ = 1,25-1,35 (m; 4H), 1,6-1,7 (m; 4H), 1,8-1,9 (m; 4H), 2,05-2,15 (m; 4H), 2,23-2,25 (s; 12H), 3,48 (s; 4H),, 3,85 (s; 4H), 4,24 (m; 4H), 5,48 (s; 1H), 7,03 (s; 4H) ppm.

### Beispiel 2

### 2-Hydroxyethyl-8-{[(4-chlor-2,6-dimethylphenyl)acetyl]amino}-1,4-dioxaspiro[4.5]decan-8-carboxylat (Bsp. XVI-1)

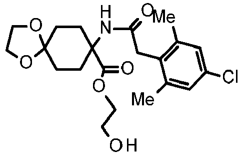

Zu einer Lösung von 1,689 g [5 mmol] 1-{[(4-Chlor-2,6-dimethylphenyl)acetyl]amino}-4-oxocyclohexancarbonsäure in 7,5 ml Chlorbenzol gibt man 2,483 g [40 mmol] Ethylenglykol und 5 Tropfen konz. Schwefelsäure. Das Reaktionsgemisch wird 4,5 Stunden auf 100 bis 105°C erhitzt und anschließend bei Raumtemperatur mit Wasser und Methylenchlorid verrührt. Man trennt die organische Phase ab, wäscht sie mit Wasser, trocknet über Natriumsulfat und engt im Vakuum ein. Man erhält 1,98 g farblosen Feststoff, der lt. HPLC 83,1% der Titelverbindung enthält (77,3% d.Th.).

### Beispiel 3

### 3-Hydroxy-2,2-dimethylpropyl-9- {[(4-chlor-2,6-dimethylphenyl)acetyl] amino} -3,3-dimethyl-1,5-dioxaspiro[5.5]undecan-9-carboxylat (Bsp. XVI-2)

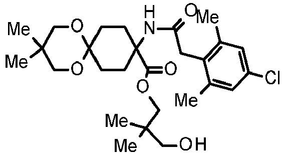

Zu einer Lösung von 5,067 g [15 mmol] 1-{[(4-Chlor-2,6-dimethylphenyl)acetyl]amino}-4-oxocyclohexancarbonsäure in 22,5 ml Chlorbenzol gibt man 12,5 g [120 mmol] 1,3-Dihydroxy-2,2-dimethylpropan und 15 Tropfen konz. Schwefelsäure. Das Reaktionsgemisch wird 4 Stunden auf 96 bis 105°C erhitzt und anschließend bei Raumtemperatur mit 25 ml Wasser und 100 ml Methylenchlorid verrührt. Man trennt die organische Phase ab, wäscht sie dreimal mit je 25 ml Wasser, trocknet über Natriumsulfat und engt im Vakuum ein. Man erhält 6,51 g farbloses Feststoff, der lt. HPLC 83,5% der Titelverbindung enthält (82,8% d.Th.).

LC/MS (ESI positiv): m/e = 510 (MH⁺, 1 ³⁵Cl).

¹H-NMR (600 MHz, CDCl₃): δ = 0,92 (s; 6H), 0,94 (s; 6H), 1,3-1,4 (m; 2H), 1,8-1,85 (m; 2H), 1,95-2,1 (m; 4H), 2,30 (s; 6H), 3,32 (s; 2H), 3,45 (d; 4H), 3,56 (s; 2H), 3,97 (s; 2H), 5,42 (s; 1H), 7,1 (s; 2H) ppm.

### Beispiel 4

### 2-Hydroxyethyl-8-[(mesitylacetyl)amino]-1,4-dioxaspiro[4.5]decan-8-carboxylat (Bsp. XVI-3)

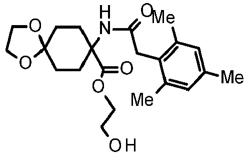

Zu einer Suspension von 2,530 g [7 mmol] 8-[(Mesitylacetyl)amino]-1,4-dioxaspiro[4.5]decan-8-carbonsäure in 19,48 g [313,8 mmol] Ethylenglykol werden bei 85°C 3 Tropfen konz. Schwefelsäure gegeben. Das Reaktionsgemisch wird 3,5 Stunden bei 100°C gerührt und dann bei Raumtemperatur in 50 ml Methylenchlorid aufgenommen. Die Methylenchloridphase wird abgetrennt und nacheinander mit gesättigter wässriger Natriumbicarbonatlösung und Wasser ausgeschüttelt, über Natriumsulfat getrocknet und im Vakuum eingeengt. Man erhält 2,90 g farblosen Feststoff, der nach HPLC 91,9% der Titelverbindung enthält. Damit ergibt sich eine Ausbeute von 93,9% d. Theorie.

LC/MS (ESI positiv): m/e = 406 (MH⁺), 811 ([2M+H]⁺).

¹H-NMR (600 MHz, CDCl₃): δ = 1,3-1,4 (m; 2H), 1,6-1,7 (m; 2H), 1,85-1,9 (m; 2H), 2,1-2,2 (m; 2H), 2,29 (s; 9H), 3,1 (s, br; 1H), 3,58 (s; 2H), 3,79 (m, br; 2H), 3,91 (s; 4H), 4,3 (m; 2H), 5,61 (s; 1H), 6,92 (s; 2H) ppm.

### Beispiel 5

### 3-Hydroxypropyl-9- { [(4-chlor-2,6-dimethylphenyl)acetyl] amino} -1,5-dioxaspiro[5.5]undecan-9-carboxylat (Bsp. XVI-4) und Propan-1,3-diyl-bis(9-{ [(4-chlor-2,6-dimethylphenyl)acetyl]amino}-1,5-dioxaspiro[5.5]undecan-9-carboxylat) (Bsp. XVII-4)

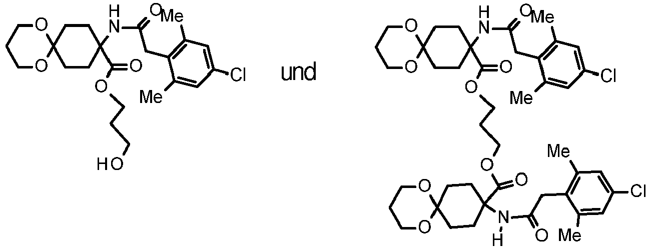

Zu einer Lösung von 5,067 g [15 mmol] 1-{[(4-Chlor-2,6-dimethylphenyl)acetyl]amino}-4-oxocyclohexancarbonsäure in 22,5 ml Chlorbenzol gibt man 9,31 g [120 mmol] 1,3-Propandiol und 15 Tropfen konz. Schwefelsäure. Das Reaktionsgemisch wird 10 Stunden auf 95-100°C erhitzt und danach bei Raumtemperatur mit Methylenchlorid und Wasser verrührt. Die organische Phase wird abgetrennt, dreimal mit Wasser gewaschen, über Natriumsulfat getrocknet und im Vakuum eingeengt. Man erhält 7,37 g harziges Öl, das lt. LC/MS 64% 3-Hydroxypropyl-9-{[(4-chlor-2,6-dimethylphenyl)acetyl]-amino}-1,5-dioxaspiro[5.5]undecan-9-carboxylat (69% d.Th.) und 5,2% Propan-1,3-diyl-bis(9-{[(4-chlor-2,6-dimethylphenyl)acetyl]amino}-1,5-dioxaspiro[5.5]undecan-9-carboxylat) (6% d. Th.) enthält.

LC/MS (ESI positiv): m/e = 454 (MH⁺, 1 ³⁵Cl), 907 ([2M+H]⁺, 2 ³⁵Cl).

LC/MS (ESI positiv): m/e = 831 (MH⁺, 2 ³⁵Cl).

### Beispiel 6

### 11-(4-Chlor-2,6-dimethylphenyl)-12-hydroxy-1,4-dioxa-9-azadispiro[4.2.4.2]tetradec-11-en-10-on (Bsp. XI-1)

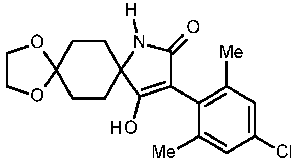

Ein Gemisch aus 1,065 g [2,50 mmol] 2-Hydroxyethyl-8-{[(4-chlor-2,6-dimethylphenyl)acetyl]amino}-1,4-dioxaspiro[4.5]decan-8-carboxylat und 0,218 g [0,28 mmol] Ethan-1,2-diyl-bis(8-{[(2,5-dimethylphenyl)acetyl]amino}-1,4-dioxaspiro[4.5]decan-8-carboxylat), hergestellt wie in Beispiel 1 beschrieben, wird in 5 ml N,N-Dimethylacetamid vorgelegt. Bei 75-80°C werden dann 1,576 g [8,75 mmol] einer 30%igen Lösung von Natriummethylat in Methanol innerhalb von 2 Minuten zugetropft. Das Reaktionsgemisch wird 55 Minuten bei 75-80°C gerührt und anschließend im Vakuum eingeengt. Der Rückstand wird in 30 ml Wasser gelöst, die Lösung filtriert und das Filtrat mit Essigsäure auf pH 4-5 gebracht. Der ausgefallene Feststoff wird abgesaugt, zweimal mit je 5 ml Wasser gewaschen und getrocknet. Man erhält 1,203 g hellbeigen Feststoff, der nach quant. NMR zu 80,7% aus der Titelverbindung besteht. Damit ergibt sich eine Ausbeute von 99% der Theorie, bezogen auf die Summe beider Eduktmoleküle.

¹H-NMR (600 MHz, d₆-DMSO): δ = 1,35-1,4 (m; 2H), 1,65-1,7 (m; 2H), 1,8-1,9 (m; 4H), 2,05-2,15 (m; 2H). 2,07 (s; 6H), 3,88 (s; 4H), 7,09 (s; 2H), 8,1 (s,br; 1H), 10,5 (s,br; 1H) ppm.

### Beispiel 7

### 3-(4-Chlor-2,6-dimethylphenyl)-4-hydroxy-11,11-dimethyl-9,13-dioxa-l-azadispiro[4.2.5.2]pentadec-3-en-2-on (Bsp. XI-2)

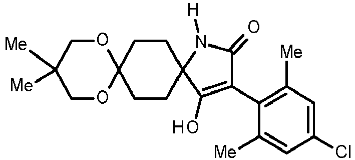

2,550 g [5 mmol] 3-Hydroxy-2,2-dimethylpropyl-9-{[(4-chlor-2,6-dimethylphenyl)acetyl]amino}-3,3-dimethyl-1,5-dioxaspiro[5.5]undecan-9-carboxylat (Beispiel 3) warden in 10 ml N,N-Dimethylacetamid vorgelegt. Bei 75-80°C werden dann 3,151 g [17,5 mmol] einer 30%igen Lösung von Natriummethylat in Methanol innerhalb von 2 Minuten zugetropft. Das Reaktionsgemisch wird 42 Minuten bei 75-80°C gerührt und anschließend im Vakuum eingeengt. Der Rückstand wird in 60 ml Wasser gelöst, die Lösung mit Essigsäure auf pH 4-5 gebracht. Der ausgefallene Feststoff wird abgesaugt, zweimal mit je 10 ml Wasser gewaschen und getrocknet. Man erhält 2,32 g hellbeigen Feststoff, der nach HPLC zu 76% aus der Titelverbindung besteht. Damit ergibt sich eine Ausbeute von 86,9% der Theorie.

LC/MS (ESI positiv): m/e = 406 (MH⁺, 1 ³⁵Cl), 811 ([2M+H]⁺, 2 ³⁵Cl).

### Beispiel 8

### 12-Hydroxy-11-mesityl-1,4-dioxa-9-azadispiro[4.2.4.2]tetradec-11-en-10-on (Bsp. XI-3)

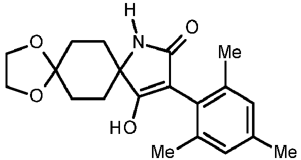

2,43 g [6,0 mmol] 2-Hydroxyethyl-8-[(mesitylacetyl)amino]-1,4-dioxaspiro[4.5]decan-8-carboxylat (Beispiel 4) werden in 12 ml N,N-Dimethylacetamid vorgelegt. Bei 75-80°C werden dann 3,78 g [21 mmol] einer 30%igen Lösung von Natriummethylat in Methanol innerhalb von 3 Minuten zugetropft. Das Reaktionsgemisch wird 6 Stunden bei 75-80°C gerührt und anschließend im Vakuum eingeengt. Der Rückstand wird in 70 ml Wasser gelöst, die Lösung mit Essigsäure auf pH 4 gebracht. Der ausgefallene Feststoff wird abgesaugt, mit 12 ml Wasser gewaschen und getrocknet. Man erhält 1,88 g hellbeigen Feststoff, der nach HPLC zu 87% aus der Titelverbindung besteht. Damit ergibt sich eine Ausbeute von 79,4% der Theorie.

LC/MS (ESI positiv): m/e = 344 (MH⁺), 687 ([2M+H]⁺).

¹H-NMR (600 MHz, d₆-DMSO): δ = 1,36-1,38 (m; 2H), 1,67-1,69 (m; 2H), 1,83-1,88 (m; 2H), 2,04 (s; 6H), 2,06-2,1 (m; 2H), 2,2 (s; 3H), 3,88 (s; 4H), 6,81 (s; 2H) ppm.

### Beispiel 9

### 3-Hydroxybutan-2-yl-8-{[(2,5-dimethylphenyl)acetyl]amino}-2,3-dimethyl-1,4-dioxaspiro[4.5]decan-8-carboxylat (Bsp. XVI-5)

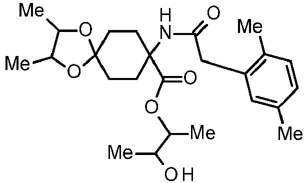

Man legt 2,87 g [7,66 mmol] 8-{[(2,5-Dimethylphenyl)acetyl]amino}-2,3-dimethyl-1,4-dioxaspiro[4.5]-decan-8-carbonsäurein 20 ml 2,3-Butandiol vor, gibt drei Tropfen konz. Schwefelsäure hinzu und rührt für 10 Stunden bei 100°C. Anschließend wird das Reaktionsgemisch bei Raumtemperatur mit Methylenchlorid verdünnt und zweimal mit Wasser ausgeschüttelt. Die organische Phase wird über Natriumsulfat getrocknet und im Vakuum eingeengt. Man erhält 4,0 g gelbliches Öl, das lt. HPLC 77,1% des Zielproduktes enthält, womit sich eine Ausbeute von 90% der Theorie ergibt.

LC/MS (ESI positiv): m/e = 448 (MH⁺), 895 ([2M+H]⁺).

### Beispiel 10

### 11-(2,5-Dimethylphenyl)-12-hydroxy-2,3-dimethyl-1,4-dioxa-9-azadispiro[4.2.4.2]tetradec-11-en-10-on (Bsp. XI-4)

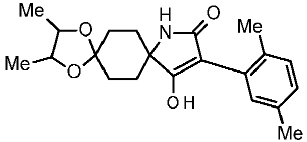

1,54 g [3,45 mmol] 3-Hydroxybutan-2-yl-8-{[(2,5-dimethylphenyl)acetyl]amino}-2,3-dimethyl-1,4-dioxaspiro[4.5]decan-8-carboxylat (Beispiel 9) werden in 7 ml N,N-Dimethylacetamid vorgelegt. Bei 75-80°C werden dann 2,17 g [12,1 mmol] einer 30%igen Lösung von Natriummethylat in Methanol innerhalb von 5 Minuten zugetropft. Das Reaktionsgemisch wird 90 Minuten bei 75-80°C gerührt und anschließend im Vakuum eingeengt. Der Rückstand wird in 40 ml Wasser gelöst, die Lösung mit Essigsäure auf pH 4-5 gebracht. Der ausgefallene Feststoff wird abgesaugt, zweimal mit je 5 ml Wasser gewaschen und getrocknet. Man erhält 0,70 g hellbeigen Feststoff, der nach HPLC zu 82,8% aus der Titelverbindung besteht. Damit ergibt sich eine Ausbeute von 47,1% der Theorie.

LC/MS (ESI positiv): m/e = 358 (MH⁺), 715 ([2M+H]⁺).

### Beispiel 11

### Kalium-8-amino-1,4-dioxaspiro[4.5]decan-8-carboxylat (Bsp. XIX-1)

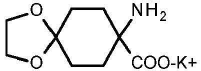

In 100 ml Wasser werden 141,4 g [0,625 mol] 9,12-Dioxa-1,3-diazadispiro[4.2.4.2]tetradecan-2,4-dion suspendiert. Man erhitzt unter einer Argonatmosphäre auf 90°C und gibt innerhalb von 15 Minuten 298,4 g [2,5 mol] Kaliumhydroxid in Form von 47%iger Kalilauge zu. Anschließend wird das Reaktionsgemisch für 24 Stunden unter Rückfluß gerührt. Unter weiterem Rühren wird auf Raumtemperatur abgekühlt und der Feststoff durch Filtration über eine Fritte isoliert. Nach Trocknen bei 60°C im Vakuum von 1 mbar erhält man 139,0 g beigen Feststoff. Quant. NMR: 88,0%. Damit errechnet sich eine Ausbeute von 81,8% der Theorie.

### Beispiel 12

### Natrium-8-amino-1,4-dioxaspiro[4.5]decan-8-carboxylat (Bsp. XIX-2)

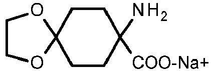

In 30 ml Wasser werden 43,4 [0,192 mol] 9,12-Dioxa-1,3-diazadispiro[4.2.4.2]tetradecan-2,4-dion suspendiert. Man erhitzt unter einer Argonatmosphäre auf 90°C und gibt innerhalb von 2 Minuten 68,3 g [0,768 mol] Natriumhydroxid in Form von 45%iger Natronlauge zu. Anschließend wird das Reaktionsgemisch für 27,5 Stunden unter Rückfluß gerührt. Unter weiterem Rühren wird auf Raumtemperatur abgekühlt und der Feststoff durch Filtration über eine Fritte isoliert. Nach Trocknen bei 60°C im Vakuum von 1 mbar erhält man 54,8 g beigen Feststoff. Quant. NMR: 72,7%. Damit errechnet sich eine Ausbeute von 93,0% der Theorie.

¹H-NMR (600 MHz, D₂O): δ = 1,56-1,6 (m, 2H), 1,69-1,75 (m, 2H), 1,75-1,82 (m, 2H), 2,0-2,04 (m, 2H), 4,06 (s, 4H) ppm.

### Beispiel 13

### Natrium-8-amino-1,4-dioxaspiro[4.5]decan-8-carboxylat (Bsp. XIX-2)

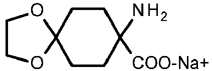

In 495 ml Wasser werden 291,8 [1,29 mol] 9,12-Dioxa-1,3-diazadispiro[4.2.4.2]tetradecan-2,4-dion suspendiert. Man erhitzt unter einer Argonatmosphäre auf 90°C und gibt innerhalb von 17 Minuten 773,3 g [8,7 mol] Natriumhydroxid in Form von 45%iger Natronlauge zu. Anschließend wird das Reaktionsgemisch für 20 Stunden unter Rückfluß gerührt. Unter weiterem Rühren wird auf Raumtemperatur abgekühlt und der Feststoff durch Filtration über eine Fritte isoliert. Nach Trocknen bei 60°C im Vakuum von 1 mbar erhält man 367,3 g beigen Feststoff. Quant. NMR: 76,3%. Damit errechnet sich eine Ausbeute von 97,1% der Theorie.

### Beispiel 14

### 1-{[(4-Chlor-2,6-dimethylphenyl)acetyl]amino}-4-oxocyclohexancarbonsäure (Bsp. XV-1)

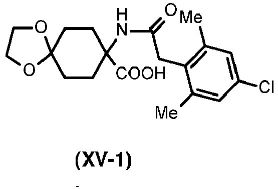

23,88 g [81,4 mmol] Kalium-8-amino-1,4-dioxaspiro[4.5]decan-8-carboxylat von 81,6% Reinheit nach quant. NMR werden bei Raumtemperatur in 65 ml Wasser vorgelegt, wobei sich ein pH-Wert von etwa 13,6 ergibt. Die Lösung wird auf 2°C abgekühlt und mit 32%iger Salzsäure auf pH 12,1 gebracht. Dann wird innerhalb von 70 Minuten bei 3 bis 5°C eine Lösung von 17,68 g [81,4 mmol] (4-Chlor-2,6-dimethylphenyl)acetylchlorid in 20 ml trockenem Tetrahydrofuran zugetropft. Gleichzeitig wird 32%ige Natronlauge in einer solchen Geschwindigkeit zugetropft, dass der pH-Wert des Reaktionsgemisches immer zwischen 11,8 und 12,5 liegt (10,93 g Verbrauch [87,4 mmol]). Man rührt dann noch weitere 60 Minuten bei 3 bis 5°C, lässt auf Raumtemperatur kommen, verdünnt mit 30 ml Wasser und stellt unter gutem Rühren den pH-Wert des Reaktionsgemisches auf etwa 1,8. Der ausgefallene Feststoff wird zweimal mit je 30 ml Wasser gewaschen und dann bei ca. 60°C im Vakuum getrocknet. Man erhält 29,31 g fast farblosen Feststoff.

Quant. NMR: 88,7%ig, womit sich eine Ausbeute von 83,6% der Theorie ergibt.

¹H-NMR (600 MHz, d₆-DMSO): δ = 1,56-1.58 (m, 2H), 1,64-1,69 (m, 2H), 1,84-1,9 (m, 2H), 2,01-2,03 (m, 2H), 2,24 (s, 6H), 3,54 (s, 2H),3,86 (s, 4H), 7,05 (s, 2H), 8,17 (s, 1H), 12 (s, br, 1H) ppm.

### Vergleichsbeispiel 1

### Methyl-8-amino-1,4-dioxaspiro[4.5]decan-8-carboxylat

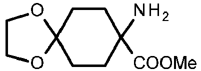

Zu einer Suspension von 64,5 g 8-Amino-1,4-dioxaspiro[4.5]decan-8-carbonsäure in 960 ml Methanol werden bei 5 bis 10°C innerhalb von 60 Minuten 57 g Thionylchlorid getropft. Man erwärmt auf 40 bis 45°c und rührt für 48 Stunden bei dieser Temperatur. Nach Abkühlen auf 5°C wird der Feststoff abgesaugt, mit 60 ml kaltem Methanol gewaschen und getrocknet. Anschließend wird der Feststoff unter Rühren in eine Lösung von 54 g Kaliumcarbonat in 220 ml Wasser eingetragen und etwa 30 Minuten verrührt. Dann wird fünfmal mit je 200 ml Methylenchlorid extrahiert. Die vereinigten organischen Phasen werden über Natriumsulfat getrocknet und bei 30°C einrotiert. Man erhält 65,8 g dunkles Öl, das lt. GC/MS nach Silylierung ca. 52,7% der Titelverbindung und 35,7% Methyl-1-amino-4,4-dimethoxycyclohexancarboxylat enthält.

### Vergleichsbeispiel 2

### Methyl-8-{[(4-chlor-2,6-dimethylphenyl)acetyl]amino} -1,4-dioxaspiro[4.5]decan-8-carboxylat

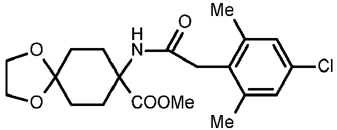

Zu einer Lösung von 33,4 g des Produktes aus Vergleichsbeispiel 1 in 490 ml trockenem Acetonitril werden bei Raumtemperatur 38 g Kaliumcarbonat gegeben. Anschließend dosiert man bei einer Temperatur von 5 bis 10°C innerhalb 1 Stunde eine Lösung von 30 g (4-Chlor-2,6-dimethylphenyl)acetylchlorid in 60 ml trockenem Acetonitril zu. Man rührt noch 2 Stunden bei 5°C und über Nacht bei Raumtemperatur, gibt das Reaktionsgemisch in etwa 2 Liter Wasser, rührt 1 Stunde bei Raumtemperatur, filtriert den ausgefallenen Feststoff ab, wäscht ihn mit 250 ml Wasser und trocknet bei 50°C im Vakuum. Man erhält 53,2 g weißen Feststoff, der lt. GC 66,4% der Titelverbindung, 18,2% Methyl-1-{[(4-chlor-2,6-dimethylphenyl)acetyl]amino}-4,4-dimethoxycyclohexancarboxylat und 12,4% Methyl-1-{ [(4-chlor-2,6-dimethylphenyl)acetyl]amino}-4-oxocyclohexancarboxylat enthält.

### Vergleichsbeispiel 3

### Methyl-8-{[(4-chlor-2,6-dimethylphenyl)acetyl]amino}-1,4-dioxaspiro[4.5]decan-8-carboxylat

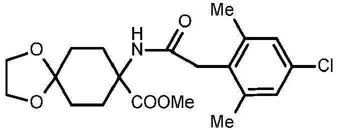

Eine Lösung von 28,0 g rohem Methyl-8-amino-1,4-dioxaspiro[4.5]decan-8-carboxylathydrochlorid, das ca. 69%ig ist und zusätzlich ca. 21% Methyl-1-amino-4,4-dimethoxycyclohexancarboxylat enthält, in 90 ml Wasser wird auf 10°C gekühlt und mit 1N Natronlauge auf einen pH-Wert von ca. 7,2 gestellt. Man gibt 10,92 g Natriumhydrogencarbonat und 100 ml Xylol hinzu und tropft anschließend innerhalb 1 Stunde bei 5 bis 10°C eine Lösung von 23,88 g (4-Chlor-2,6-dimethylphenyl)acetylchlorid in 27 ml Xylol zu. Danach rührt man weitere 30 Minuten bei 5 bis 10°C, erwärmt dann auf 65°C und rührt 1 Stunde bei dieser Temperatur. Dabei wird der pH-Wert durch Zugabe von 1N Natronlauge bei 7 gehalten. Nach Abkühlen auf Raumtemperatur wird der ausgefallene Feststoff abgesaugt und nacheinander mit 25 ml Xylol und zweimal je 25 ml Petrolether gewaschen. Nach Trocknung im Vakuum bei 50°C erhält man einen beigen Feststoff, der lt. HPLC-Analyse 78,8% der Titelverbindung, 6,6% Methyl-1-{[(4-chlor-2,6-dimethylphenyl)acetyl] amino} -4,4-dimethoxycyclohexancarboxylat und 11,1 % Methyl-1- {[(4-chlor-2,6-dimethylphenyl)acetyl] amino} -4-oxocyclohexancarboxylat enthält.

### Vergleichsbeispiel 4

### 11 -(4-Chlor-2,6-dimethylphenyl)- 12-hydroxy-1,4-dioxa-9-azadispiro[4.2.4.2] tetradec-11 -en- 10-on

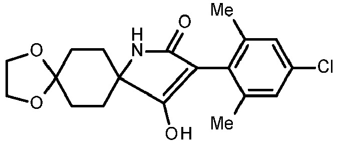

Eine Lösung von 42,0 g rohem Methyl-8-amino-1,4-dioxaspiro[4.5]decan-8-carboxylathydrochlorid, das ca. 69%ig ist und zusätzlich ca. 21% Methyl-1-amino-4,4-dimethoxycyclohexancarboxylat enthält, in 135 ml Wasser wird auf 10°C gekühlt und mit 1 N Natronlauge auf einen pH-Wert von ca. 7,2 gestellt. Man gibt 16,4 g Natriumhydrogencarbonat und 150 ml Xylol hinzu und tropft anschließend innerhalb 1 Stunde bei 5 bis 10°C eine Lösung von 29,31 g (4-Chlor-2,6-dimethylphenyl)acetylchlorid in 36 ml Xylol zu. Danach rührt man weitere 30 Minuten bei 5 bis 10°C, erwärmt dann auf 65°C und rührt 1 Stunde bei dieser Temperatur. Dabei wird der pH-Wert durch Zugabe von 1 N Natronlauge bei 7 gehalten. Anschließend wird das Wasser durch azeotrope Destillation am Wasserabscheider im Vakuum von ca. 250 bis 110 mbar entfernt. Man läßt den Sumpf auf etwa 50°C abkühlen und setzt dann 110 ml N,N-Dimethylacetamid zu. Anschließend destilliert man das Xylol im leichten Vakuum bei einem Siedepunkt von etwa 70°C ab. Man kühlt danach auf 50°C ab und tropft innerhalb von 10 Minuten 36,47 g 30%ige methanolische Natriummethylatlösung zu. Man erwärmt auf 70°C und destilliert innerhalb von etwa 2 Stunden im leichten Vakuum das Methanol ab. Anschließend wird im Vakuum das N,N-Dimethylacetamid weitgehend abdestilliert. Der Rückstand wird in 500 ml Wasser aufgenommen und die Lösung durch Zugabe von Eisessig auf einen pH-Wert von etwa 5 gebracht. Der ausgefallene Feststoff wird abgesaugt, zweimal mit je 80 ml Wasser gewaschen und bei 50°C im Vakuum getrocknet. Man erhält 51,95 g beigen Feststoff, der lt. LC/MS-Analyse 71,3% der Titelverbindung, 11,1% 3-(4-Chlor-2,6-dimethylphenyl)-4-hydroxy-8,8-dimethoxy-1-azaspiro[4.5]dec-3-en-2-on und 1,9% 3-(4-Chlor-2,6-dimethylphenyl)-4-hydroxy-1-azaspiro[4.5]dec-3-en-2,8-dion enthält.

## Patentansprüche

1. Verfahren zur Herstellung von Verbindungen der Formel (XI), 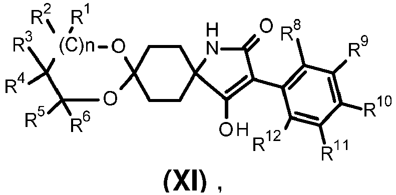
in welcher
R¹, R², R³, R⁴, R⁵, R⁶ unabhängig voneinander für Wasserstoff, Methyl, Ethyl oder Phenyl stehen,
R⁸, R⁹, R¹⁰, R¹¹, R¹² unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl stehen
und
n für 0 oder 1 steht,
**dadurch gekennzeichnet, dass** Verbindungen der Formel (III) 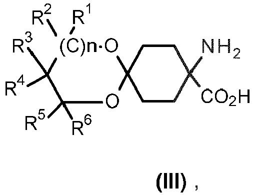
in welcher n und R¹ bis R⁶ die oben angegebenen Bedeutungen haben,
in Gegenwart einer Base mit Verbindungen der Formel (VII) 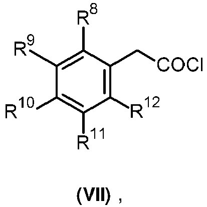
in welcher R⁸ bis R¹² die oben angegebenen Bedeutungen haben,
zu Verbindungen der Formel (XV) 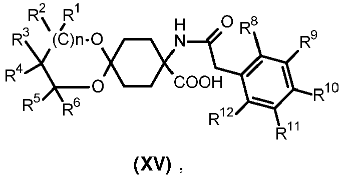
in welcher n, R¹ bis R⁶ und R⁸ bis R¹² die oben angegebenen Bedeutungen haben,
umsetzt;
daraufhin die Verbindungen der Formel (XV) mit Verbindungen der Formel (XIV)
HO-(CR¹R²)ₙ-CR³R⁴-CR⁵R⁶-OH (XIV),
in welcher n und R¹ bis R⁶ die oben angegebenen Bedeutungen haben und im konkreten Einzelfall mit denjenigen in der Verbindung der allgemeinen Formel (XV) identisch sind,
in Gegenwart einer Säure als Katalysator zu den Verbindungen der Formeln (XVI) und (XVII) 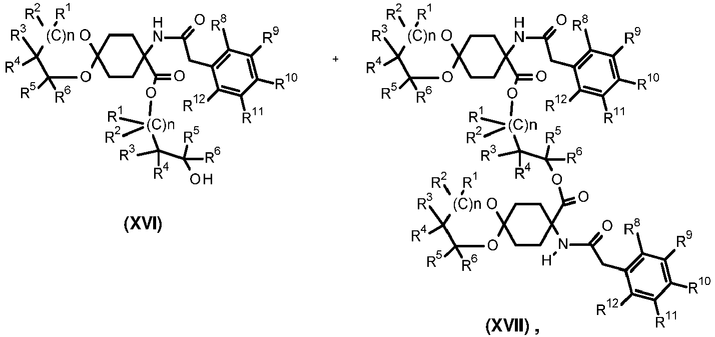
in welchen n, R¹ bis R⁶ und R⁸ bis R¹² die oben angegebenen Bedeutungen haben und sowohl n als auch die sich entsprechenden Reste R¹ bis R⁶ und R⁸ bis R¹² an beiden Positionen der Verbindungen der Formeln (XVI) oder (XVII) identisch sind,
verestert;
daraufhin die Verbindungen der Formeln (XVI) und (XVII), in denen n, R¹ bis R⁶ und R⁸ bis R¹² die oben angegebenen Bedeutungen haben,
durch Reaktion mit einer starken Base zu den Verbindungen der Formel (XI) 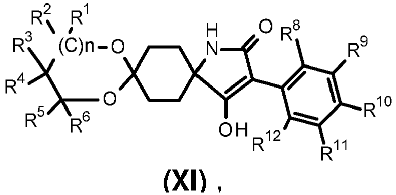
in welcher n, R¹ bis R⁶ und R⁸ bis R¹² die oben angegebenen Bedeutungen haben,
umsetzt.

2. Verfahren gemäß Anspruch 1, wobei
R¹ bis R⁶ unabhängig voneinander für Wasserstoff, Methyl oder Ethyl stehen,
R⁸ bis R¹² unabhängig voneinander für Wasserstoff, Methyl, Ethyl, Fluor, Chlor, Methoxy, Ethoxy, Trifluormethoxy oder gegebenenfalls durch Methyl, Ethyl, Methoxy, Ethoxy, Fluor, Chlor oder Brom substituiertes Phenyl stehen,
n für 0 oder 1 steht.

3. Verfahren gemäß Anspruch 1, wobei
R¹ bis R⁶ unabhängig voneinander für Wasserstoff oder Methyl stehen,
R⁸ bis R¹² unabhängig voneinander für Wasserstoff, Methyl, Ethyl, Fluor, Chlor, Methoxy, Ethoxy oder gegebenenfalls durch Methyl, Ethyl, Methoxy, Ethoxy, Fluor oder Chlor substituiertes Phenyl stehen,
n für 0 oder 1 steht.

4. Verfahren zur Herstellung von Verbindungen der Formeln (XVI) und (XVII) 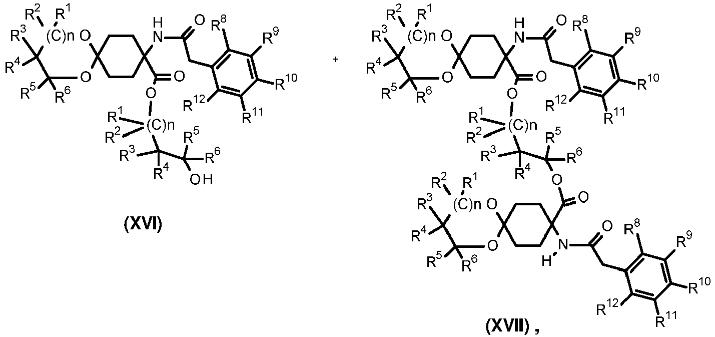 in welcher
R¹ bis R⁶ und R⁸ bis R¹² die oben angegebenen Bedeutungen haben und die sich entsprechenden Reste R¹ bis R⁶ und R⁸ bis R¹² an beiden Positionen der Verbindungen der allgemeinen Formeln (XVI) oder (XVII) identisch sind, und
n für 0 oder 1 steht und an beiden Positionen der Verbindungen der Formeln (XVI) oder (XVII) identisch ist,
**dadurch gekennzeichnet, dass** Verbindung der Formel (XVIII) 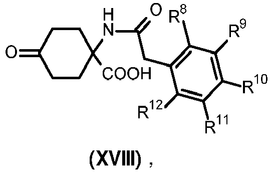
in welcher R⁸ bis R¹² die oben angegebenen Bedeutungen haben,
mit Verbinduungen der Formel (XIV)
HO-(CR¹R²)ₙ-CR³R⁴-CR⁵R⁶-OH (XIV),
in welcher n und R¹ bis R⁶ die oben angegebenen Bedeutungen haben und im konkreten Einzelfall mit denjenigen in den Verbindung der Formeln (XVI) oder (XVII) identisch sind,
in Gegenwart einer Säure als Katalysator umsetzt.

5. Verbindung der Formel (XV-1)

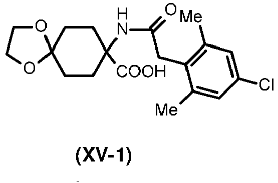

6. Verbindungen der Formeln (XVI) und (XVII) 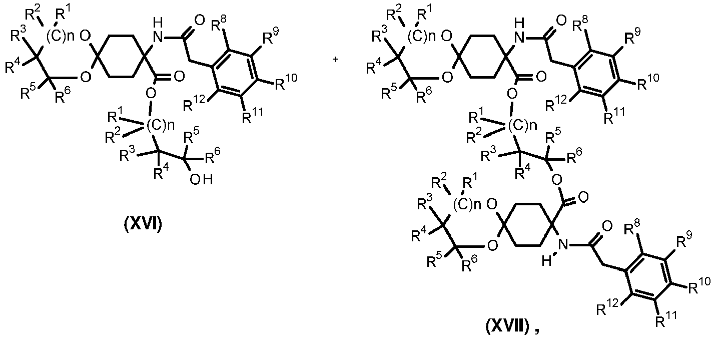 in welcher
R¹ bis R⁶ unabhängig voneinander für Wasserstoff, Methyl, Ethyl oder Phenyl stehen und an beiden Positionen der Verbindungen der Formeln (XVI) oder (XVII) identisch sind,
n für 0 oder 1
steht und an beiden Positionen der Verbindungen der Formeln (XVI) oder (XVII) identisch ist, und
R⁸ bis R¹² unabhängig voneinander für Wasserstoff, Methyl, Ethyl, Fluoralkyl mit ein oder 2 C-Atomen und ein bis fünf Fluoratomen, Halogen, Methoxy, Ethoxy, Trifluormethoxy oder gegebenenfalls durch Methyl, Ethyl, Methoxy, Ethoxy oder Halogen substituiertes Phenyl stehen und an beiden Positionen der Verbindungen der Formeln (XVI) oder (XVII) identisch sind.

7. Verbindungen der Formeln (XVI) und (XVII) gemäß Anspruch 6, in welcher
R³ bis R⁶ unabhängig voneinander für Wasserstoff oder Methyl stehen und an beiden Positionen der Verbindungen der Formeln (XVI) oder (XVII) identisch sind,
n für 0 steht
und
R⁸ bis R¹² unabhängig voneinander für Wasserstoff, Methyl, oder Chlor stehen und an beiden Positionen der Verbindungen der Formeln (XVI) oder (XVII) identisch sind.

8. Verbindungen der Formeln (XVI) und (XVII) gemäß Anspruch 6,
in welcher
R³ bis R⁶ für Wasserstoff stehen
n für 0 steht und
R⁸ für Methyl steht,
R⁹ für Wasserstoff steht,
R¹⁰ für Chlor steht,
R¹¹ für Wasserstoff steht,
R¹² für Methyl steht.

## Claims

1. Process for preparing compounds of the formula (XI) 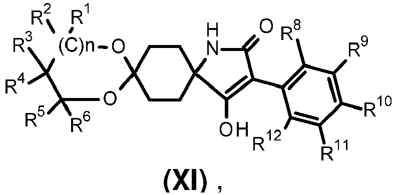 where
R¹, R², R³, R⁴, R⁵, R⁶ independently of one another represent hydrogen, methyl, ethyl or phenyl, R⁸, R⁹, R¹⁰, R¹¹, R¹² independently of one another represent hydrogen, methyl, ethyl, fluoroalkyl having one or 2 carbon atoms and one to five fluorine atoms, halogen, methoxy, ethoxy, trifluoromethoxy or optionally methyl-, ethyl-, methoxy-, ethoxy- or halogen-substituted phenyl
and
n represents 0 or 1,
**characterized in that** compounds of the formula (III) 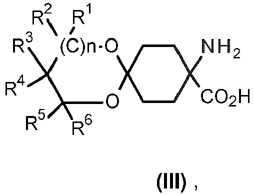
where n and R¹ to R⁶ have the meanings given above,
are reacted in the presence of a base with compounds of the formula (VII) 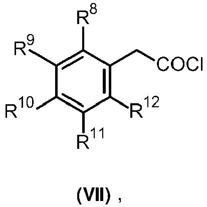
where R⁸ to R¹² have the meanings given above, to give compounds of the formula (XV) 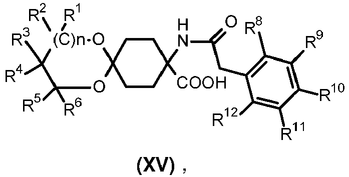
where n, R¹ to R⁶ and R⁸ to R¹² have the meanings given above;
the compounds of the formula (XV) are then esterified with compounds of the formula (XIV)
HO - (CR¹R²)ₙ - CR³R⁴ - CR⁵R⁶ -OH **(XIV),**
where n and R¹ to R⁶ have the meanings given above and are, in the concrete individual case, identical to those in the compound of the general formula (XV),
in the presence of an acid as catalyst, to give the compounds of the formulae (XVI) and (XVII) 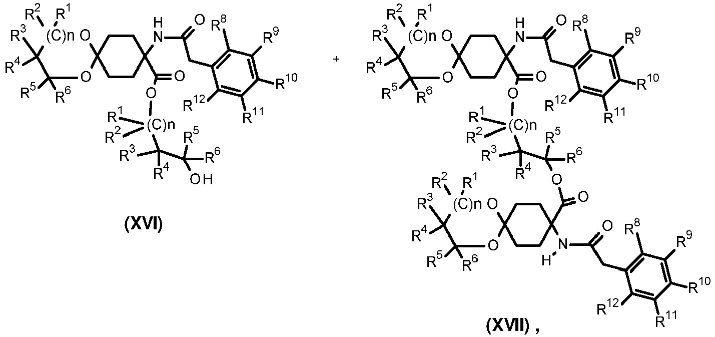
where n, R¹ to R⁶ and R⁸ to R¹² have the meanings given above and both n and the corresponding radicals R¹ to R⁶ and R⁸ to R¹² at the two positions of the compounds of the formulae (XVI) or (XVII) are identical;
the compounds of the formulae (XVI) and (XVII) in which n, R¹ to R⁶ and R⁸ to R¹² have the meanings given above,
are then converted by reaction with a strong base into the compounds of the formula (XI) 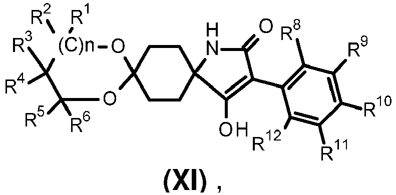
where n, R¹ to R⁶ and R⁸ to R¹² have the meanings given above.

2. Process according to Claim 1, where
R¹ to R⁶ independently of one another represent hydrogen, methyl or ethyl,
R⁸ to R¹² independently of one another represent hydrogen, methyl, ethyl, fluorine, chlorine, methoxy, ethoxy, trifluoromethoxy or optionally methyl-, ethyl-, methoxy-, ethoxy-, fluorine-, chlorine- or bromine-substituted phenyl,
n represents 0 or 1.

3. Process according to Claim 1, where
R¹ to R⁶ independently of one another represent hydrogen or methyl,
R⁸ to R¹² independently of one another represent hydrogen, methyl, ethyl, fluorine, chlorine, methoxy, ethoxy or optionally methyl-, ethyl-, methoxy-, ethoxy-, fluorine- or chlorine-substituted phenyl,
n represents 0 or 1.

4. Process for preparing compounds of the formulae (XVI) and (XVII) 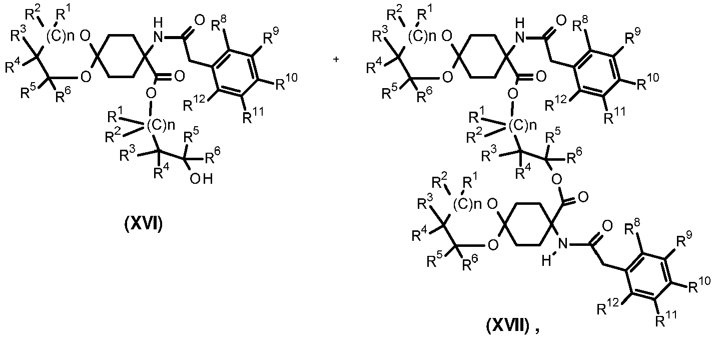 where
R¹ to R⁶ and R⁸ to R¹² have the meanings given above and the corresponding radicals R¹ to R⁶ and R⁸ to R¹² at the two positions of the compounds of the general formulae (XVI) or (XVII) are identical and
n represents 0 or 1 and is identical at the two positions of the compounds of the formulae (XVI) or (XVII),
**characterized in that** compound of the formula (XVIII) 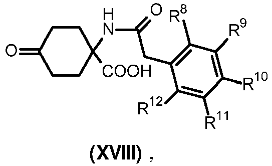
where R⁸ to R¹² have the meanings given above, is reacted with compounds of the formula (XIV)
HO - (CR¹R²)ₙ -CR³R⁴- CR⁵R⁶- OH **(XIV) ,**
where n and R¹ to R⁶ have the meanings given above and are, in the concrete individual case, identical to those in the compound of the formulae (XVI) or (XVII),
in the presence of an acid as catalyst.

5. Compound of the formula (XV-1)

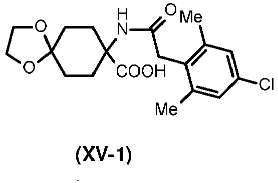

6. Compounds of the formulae (XVI) and (XVII) 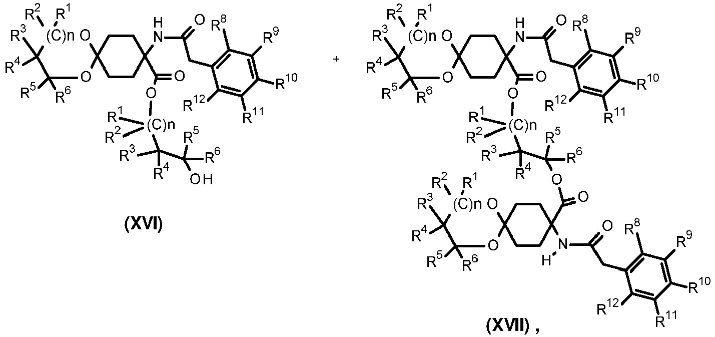 where
R¹ to R⁶ independently of one another represent hydrogen, methyl, ethyl or phenyl and are identical at the two positions of the compounds of the formulae (XVI) or (XVII),
n represents 0 or 1
and is identical at the two positions of the compounds of the formulae (XVI) or (XVII),
and
R⁸ to R¹² independently of one another represent hydrogen, methyl, ethyl, fluoroalkyl having one or 2 carbon atoms and one to five fluorine atoms, halogen, methoxy, ethoxy, trifluoromethoxy or optionally methyl-, ethyl-, methoxy-, ethoxy- or halogen-substituted phenyl
and are identical at the two positions of the compounds of the formulae (XVI) or (XVII).

7. Compounds of the formulae (XVI) and (XVII) according to Claim 6,
where
R³ to R⁶ independently of one another represent hydrogen or methyl and are identical at the two positions of the compounds of the formulae (XVI) or (XVII),
n represents 0
and
R⁸ to R¹² independently of one another represent hydrogen, methyl or chlorine and are identical at the two positions of the compounds of the formulae (XVI) or (XVII).

8. Compounds of the formulae (XVI) and (XVII) according to Claim 6,
where
R³ to R⁶ represent hydrogen,
n represents 0 and
R⁸ represents methyl,
R⁹ represents hydrogen,
R¹⁰ represents chlorine,
R¹¹ represents hydrogen,
R¹² represents methyl.

## Revendications

1. Procédé de préparation de composés de formule (XI) 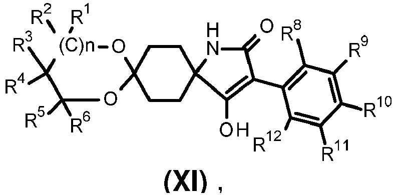 dans laquelle
R¹, R², R³, R⁴, R⁵, R⁶ représentent, indépendamment les uns des autres, hydrogène, méthyle, éthyle ou phényle,
R⁸, R⁹, R¹⁰, R¹¹, R¹² représentent, indépendamment les uns des autres, hydrogène, méthyle, éthyle, fluoroalkyle comprenant un ou 2 atomes de carbone et un à cinq atomes de fluor, halogène, méthoxy, éthoxy, trifluorométhoxy ; ou phényle le cas échéant substitué par méthyle, éthyle, méthoxy, éthoxy ou halogène et
n représente 0 ou 1,
**caractérisé en ce qu'**on transforme des composés de formule (III) 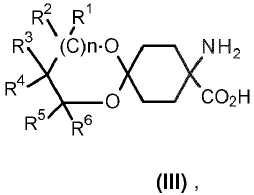
dans laquelle n et R¹ à R⁶ présentent les significations indiquées ci-dessus, en présence d'une base, avec des composés de formule (VII) 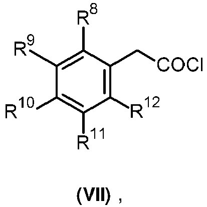
dans laquelle R⁸ à R¹² présentent les significations indiquées ci-dessus, en composés de formule (XV) 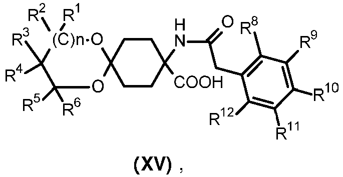
dans laquelle n, R¹ à R⁶ et R⁸ à R¹² ont les significations indiquées ci-dessus ;
puis on estérifie les composés de formule (XV) avec des composés de formule (XIV)
HO-(CR¹R²)ₙ-CR³R⁴-CR⁵R⁶-OH (XIV) ,
dans laquelle n et R¹ à R⁶ présentent les significations indiquées ci-dessus et sont, dans le cas individuel concret, identiques à ceux dans le composé de formule générale (XV)
en présence d'un acide en tant que catalyseur en composés des formules (XVI) et (XVII) 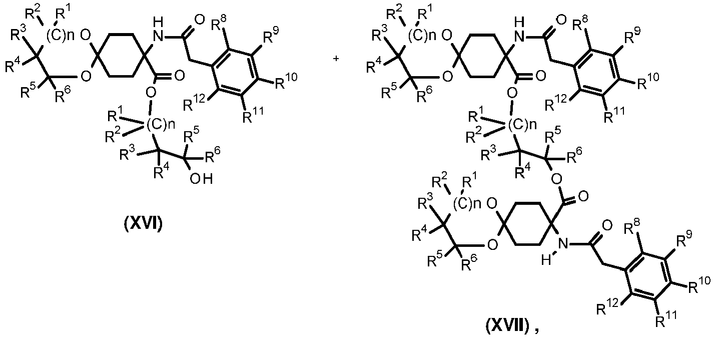
dans lesquelles n, R¹ à R⁶ et R⁸ à R¹² présentent les significations indiquées ci-dessus et tant n que les radicaux R¹ à R⁶ et R⁸ à R¹² qui se correspondent sont identiques dans les deux positions des composés des formules (XVI) ou (XVII) ;
puis on transforme les composés des formules (XVI) et (XVII), dans lesquelles n et R¹ à R⁶ et R⁸ à R¹² présentent les significations indiquées ci-dessus,
par réaction avec une base forte en composés de formule (XI) 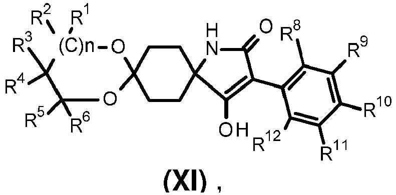
dans laquelle n, R¹ à R⁶ et R⁸ à R¹² ont les significations indiquées ci-dessus.

2. Procédé selon la revendication 1, où
R¹ à R⁶⁶ représentent, indépendamment les uns des autres, hydrogène, méthyle ou éthyle
R⁸ à R¹² représentent, indépendamment les uns des autres, hydrogène, méthyle, éthyle, fluor, chlore, méthoxy, éthoxy, trifluorométhoxy ; ou phényle le cas échéant substitué par méthyle, éthyle, méthoxy, éthoxy, fluor, chlore ou brome,
n représente 0 ou 1.

3. Procédé selon la revendication 1, où
R¹ à R⁶ représentent, indépendamment les uns des autres, hydrogène ou méthyle,
R⁸ à R¹² représentent, indépendamment les uns des autres, hydrogène, méthyle, éthyle, fluor, chlore, méthoxy, éthoxy ; ou phényle le cas échéant substitué par méthyle, éthyle, méthoxy, éthoxy, fluor ou chlore,
n représente 0 ou 1.

4. Procédé de préparation de composés des formules (XVI) et (XVII) 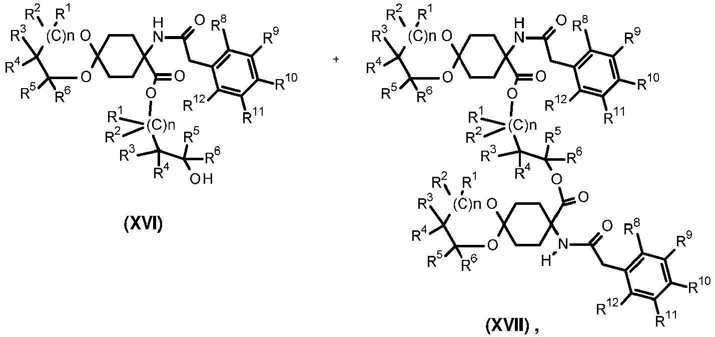 dans lesquelles
R¹ à R⁶ et R⁸ à R¹² présentent les significations indiquées ci-dessus et les radicaux R¹ à R⁶ et R⁸ à R¹² qui se correspondent sont identiques dans les deux positions des composés des formules générales (XVI) ou (XVII) et
n représente 0 ou 1 et est identique dans les deux positions des composés des formules (XVI) ou (XVII),
**caractérisé en ce qu'**on transforme le composé de formule (XVIII) 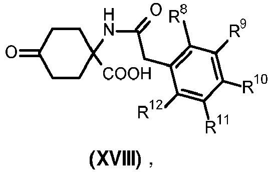
dans laquelle R⁸ à R¹² présentent les significations indiquées ci-dessus, avec des composés de formule (XIV)
HO-(CR¹R²)ₙ-CR³R⁴-CR⁵R⁶-OH **(XIV)** ,
dans laquelle n et R¹ à R⁶ présentent les significations indiquées ci-dessus et sont, dans le cas individuel concret, identiques à ceux dans le composé des formules générales (XVI) ou (XVIII)
en présence d'un acide en tant que catalyseur.

5. Composé de formule (XV-1)

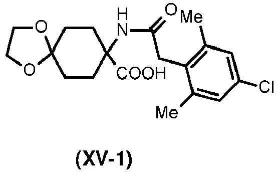

6. Composés des formules (XVI) et (XVII) 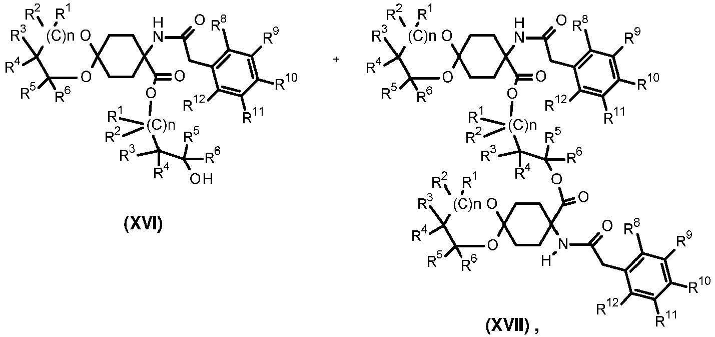 dans lesquelles
R¹ à R⁶ représentent, indépendamment les uns des autres, hydrogène, méthyle, éthyle ou phényle et sont identiques dans les deux positions des composés des formules (XVI) ou (XVII),
n représente 0 ou 1 et est identique dans les deux positions des composés des formules (XVI) ou (XVII) et
R⁸ à R¹² représentent, indépendamment les uns des autres, hydrogène, méthyle, éthyle, fluoroalkyle comprenant un ou 2 atomes de carbone et un à cinq atomes de fluor, halogène, méthoxy, éthoxy, trifluorométhoxy ; ou phényle le cas échéant substitué par méthyle, éthyle, méthoxy, éthoxy ou halogène et sont identiques dans les deux positions des composés des formules (XVI) ou (XVII).

7. Composés des formules (XVI) et (XVII) selon la revendication 6, où
R³ à R⁶ représentent, indépendamment les uns des autres, hydrogène ou méthyle et sont identiques dans les deux positions des composés des formules (XVI) ou (XVII),
n représente 0 et
R⁸ à R¹² représentent, indépendamment les uns des autres, hydrogène, méthyle ou chlore et sont identiques dans les deux positions des composés des formules (XVI) ou (XVII).

8. Composés des formules (XVI) et (XVII) selon la revendication 6, où
R³ à R⁶ représentent hydrogène,
n représente 0 et
R⁸ représente méthyle,
R⁹ représente hydrogène,
R¹⁰ représente chlore,
R¹¹ représente hydrogène,
R¹² représente méthyle.
